# DEMANDE DE BREVET EUROPEEN

(11) **EP 2 775 433 A1**
(43) Date de publication de la demande: **10.09.2014**
(21) Numéro de dépôt: 14158121.5
(22) Date de dépôt: 06.03.2014
(51) Int. Cl.: G06Q 10/08

(54) **Dispositif pour sélectionner des lentilles de contact au sein d'un meuble agencé pour recevoir une pluralité de lentilles de contact et procédé associé**

(30) Priorité: 06.03.2013 FR 1352030
(71) Demandeur: Ophtalmic Compagnie, 93420 Villepinte (FR)
(72) Inventeur: Tamsot, Charli, 91440 Jesrusalem (IL)
(74) Mandataire: Reboussin, Yohann Mickaël Noël

(57) **Abrégé**

L'invention concerne un dispositif (100) pour sélectionner des lentilles de contact au sein d'un meuble (1) agencé pour recevoir une pluralité de lentilles de contact (50), caractérisé en ce qu'il comprend : - un moyen (4) pour stocker des données basées sur les mesures issues d'un auto-réfractomètre (20); - un moyen (5) de traitement de données associé à un module de calcul (51) pour identifier une lentille de contact sur la base d'une part, des données de issues de l'auto-réfractomètre (20) qui ont été préalablement stockées dans le moyen de stockage (4) et d'autre part, de données complémentaires relatives à la lentille de contact à identifier qui ont été préalablement entrées par l'utilisateur, ledit moyen (5) de traitement de données associé audit module de calcul (51) étant également agencé pour identifier l'emplacement, au sein du meuble (1), de cette lentille.

Elle concerne également un procédé associé.

## Description

L'invention concerne un dispositif pour sélectionner des lentilles de contact au sein d'un meuble, ainsi qu'un procédé associé.

Lorsqu'un ophtalmologiste examine un patient, il utilise un auto-réfractomètre pour déterminer la nature de l'amétropie (défaut visuel) de son patient. Cet auto-réfractomètre est généralement connecté à l'ordinateur personnel de l'ophtalmologiste, ordinateur qui comporte un logiciel capable de traiter les données issues du réfractomètre et permettant notamment l'affichage des mesures prises par l'auto-réfractomètre. Un exemple de logiciel largement utilisé à cet effet est le logiciel StudioVision proposé par la société RealVision.

Typiquement, les différentes amétropies sont les suivantes : myopie, hypermétropie, astigmatisme, presbytie.

Les mesures réalisées par l'auto-réfractomètre sont des mesures objectives. Elles fournissant notamment pour chaque oeil du patient, via le logiciel mentionné ci-dessus, les données classiques suivantes :
- sphère (selon son signe, cette mesure est relative au degré de myopie ou d'hypermétropie de l'oeil considéré du patient),
- cylindre (mesure relative au degré d'astigmatisme),
- axe (mesure relative à l'axe d'astigmatisme),
- addition (mesure relative à la correction de la presbytie par lentilles progressives ; donne des valeurs à ajouter à la vision de loin pour une correction en vision de près),
- rayon (s) (mesure(s) relatives au rayon de courbure de la cornée de l'oeil du patient. En général, deux prises de rayons de courbure de la cornée sont effectuées pour déterminer la kératométrie maximale et la kératométrie minimale), et généralement, les données additionnelles suivantes :
- axe du ou de chaque rayon de courbure de la cornée considéré dans la mesure.

Sur la base de ces mesures objectives, l'ophtalmologiste effectue généralement une évaluation subjective visant à adapter les données objectives fournies par l'auto-réfractomètre. Cette adaptation vise à prendre en compte le mode de vie journalier de son patient, tels que son exposition quotidienne à la lumière, la nature de son travail ou sa pratique sportive.

L'ophtalmologiste détermine ensuite les caractéristiques des lentilles de contact devant être proposées à son patient.

A cet effet, l'ophtalmologiste prend en compte la nature de l'amétropie de son patient, déterminée par l'auto-réfractomètre pour choisir la forme de la lentille (par exemple sphérique, torique, multifocale) la mieux adaptée au patient.

Il prend en compte la nature du matériau formant la lentille de contact (par exemple silicone hydrogel, hydrogel, rigide).

Il prend enfin en compte la fréquence de port (par exemple lentille journalière, mensuelle, bi-mensuelle ou trimestrielle) qu'il estime être la mieux adaptée pour son patient.

Il prend également en compte les mesures réalisées par son auto-réfractomètre et, éventuellement, sa correction subjective.

Il convient de noter que lorsqu'une évaluation subjective est effectuée sur la base des mesures objectives réalisées par l'auto-réfractomètre (ce qui est souvent le cas), l'ophtalmologiste doit, par lui-même, réaliser des corrections des mesures réalisées par l'auto-réfractomètre.

A cet effet, il fait appel à son expérience et/ou à des tableaux d'équivalence bien connus de l'homme du métier.

Pour certaines lentilles de contact, cette correction est assez aisée et l'ophtalmologiste effectue un choix en faisant appel à son expérience.

Cependant, ce n'est pas le cas de toutes les lentilles de contact. Par exemple, cette correction est plus difficile à déterminer lorsque des lentilles toriques doivent être proposées au patient. Dans ce dernier cas, l'ophtalmologiste a besoin d'un peu de temps pour faire son choix dans la correction qu'il doit finalement choisir, en s'appuyant sur des tableaux d'équivalence.

Une perte de temps peut donc être occasionnée, tout comme une erreur dans sa correction subjective.

Une fois que l'ensemble des caractéristiques des lentilles de contact a été déterminé, l'ophtalmologiste choisit dans son stock les lentilles concernées. A cet effet, l'ophtalmologiste dispose généralement de plusieurs meubles de stockage de différents fournisseurs.

Au bout d'un certain temps, l'ophtalmologiste doit alors passer commande des lentilles de contact qu'il n'a plus dans son stock afin qu'un salarié de son fournisseur réalise un réassort de son stock.

Cela génère une perte de temps pour l'ophtalmologiste. Par ailleurs, une erreur dans la nature des lentilles de contact devant être fournies peut être engendrée.

Si l'ophtalmologiste ne passe pas commande, le passage régulier de son fournisseur peut permettre de réaliser un réassort, mais qui n'est généralement pas adéquat car le fournisseur n'a aucune idée des lentilles de contact manquantes.

Le réassort de lentilles de contact disponibles chez l'ophtalmologiste (lentilles d'essais) pose donc problème, tant pour l'ophtalmologiste que pour son fournisseur.

Un objectif de l'invention est de proposer un dispositif et/ou un procédé qui ne présente pas l'un au moins des inconvénients précités.

A cet effet, l'invention propose un dispositif pour sélectionner des lentilles de contact au sein d'un meuble agencé pour recevoir une pluralité de lentilles de contact, caractérisé en ce qu'il comprend :
- un moyen pour stocker des données basées sur les mesures issues d'un auto-réfractomètre ;
- un moyen de traitement de données associé à un module de calcul pour identifier une lentille de contact sur la base d'une part, des données de issues de l'auto-réfractomètre qui ont été préalablement stockées dans le moyen de stockage et d'autre part, de données complémentaires relatives à la lentille de contact à identifier qui ont été préalablement entrées par l'utilisateur, ledit moyen de traitement de données associé audit module de calcul étant également agencé pour identifier l'emplacement, au sein du meuble, de cette lentille.

Le dispositif selon l'invention pourra également comprendre l'une au moins des caractéristiques suivantes, prises seules ou en combinaison :
- un moyen pour identifier les informations relatives au contenu d'un boîtier comportant la lentille de contact dont l'emplacement dans le meuble a été identifié, lequel est associé à un module de prise en compte de ces données au sein du dispositif ;
- un moyen pour transmettre à distance, par exemple par l'intermédiaire du réseau Internet, les informations relatives à la lentille de contact dont le boîtier a été identifié et, un serveur informatique distant capable de communiquer avec le moyen pour transmettre à distance ces informations relatives à la lentille de contact, le serveur étant agencé pour comptabiliser les lentilles de contact sorties du meuble ;
- le module de calcul est implanté dans le serveur informatique distant ;
- le module de calcul est logé dans le meuble ;
- un moyen d'affichage pour afficher lesdites données complémentaires relatives à la lentille de contact parmi lesquelles des données, à fournir par un utilisateur, concernant la forme de la lentille, la nature du matériau formant la lentille et la fréquence de port de cette lentille ;
- un moyen de communication avec un ordinateur personnel de l'utilisateur stockant les données issues de l'auto-réfractomètre, telle qu'une liaison Bluetooth® ;
- le meuble comporte plusieurs tiroirs, chaque tiroir étant agencé pour recevoir plusieurs séries de lentilles de contact d'un même modèle pour lequel chaque série de lentilles de contact se distingue d'une autre série de lentilles de contact par la puissance de la correction ophtalmique ;
- le meuble se présente sous la forme d'un distributeur automatique de boîtiers de lentilles de contact ;
- le meuble distributeur comprend un moyen pour prélever automatiquement le boîtier comportant la lentille de contact de son emplacement, par exemple une gâchette électrique capable d'extraire ledit boîtier auquel est associée une gouttière capable de transférer ledit boîtier vers une sortie du meuble ;
- le moyen pour identifier les informations relatives au contenu d'un boîtier comportant la lentille de contact, par exemple un lecteur RFID ou de code-barres, est disposé à l'intérieur du meuble, avantageusement au niveau de la sortie du meuble ;
- le meuble distributeur comporte une entrée, se présentant par exemple sous la forme d'une fente, pour insérer un boîtier de lentilles de contact ;
- le meuble distributeur comprend un autre moyen pour identifier les informations relatives au contenu d'un boîtier comportant la lentille de contact, avantageusement disposé à l'intérieur du meuble au niveau de l'entrée de ce meuble ;
- le meuble distributeur comprend des moyens pour remettre le boîtier à son emplacement ;
- le moyen de traitement de données associé au module de calcul est également agencé pour déterminer les caractéristiques d'une nouvelle lentille à partir des caractéristiques d'une lentille préalablement identifiée et de données complémentaires mesurées par l'utilisateur sur le patient muni de ladite lentille préalablement identifiée, ces données complémentaires comportant au moins une mesure de surréfraction, ledit moyen de traitement de données associé audit module de calcul étant également agencé pour identifier l'emplacement, au sein du meuble, de cette nouvelle lentille ;
- la lentille préalablement identifiée étant une lentille sphérique, le moyen de traitement de données associé au module de calcul est agencé pour déterminer la sphère, le cylindre et l'axe de la nouvelle lentille à partir des caractéristiques de la lentille préalablement identifiée et d'une mesure de surréfraction réalisée sur le patient muni de cette lentille préalablement identifiée ;
- la lentille préalablement identifiée étant une lentille torique, le moyen de traitement de données associé au module de calcul est agencé pour déterminer la sphère, le cylindre et l'axe de la nouvelle lentille à partir des caractéristiques de la lentille préalablement identifiée et de mesures de surréfraction et de rotation réalisées par l'utilisateur sur le patient muni de cette lentille préalablement identifiée ;
- la lentille préalablement identifiée étant une lentille progressive, le moyen de traitement de données associé au module de calcul est agencé pour déterminer la sphère et/ou l'addition de la nouvelle lentille à partir des caractéristiques de la lentille préalablement identifiée, notamment l'addition, de l'âge du patient et, de mesures de surréfraction, duochrome, d'acuité visuelle en vision de près et de loin et de la détermination de l'oeil adelphe ou dominant du patient réalisées par l'utilisateur sur le patient muni de cette lentille préalablement identifiée.

A cet effet, l'invention propose également un procédé de sélection de lentilles de contact au sein d'un meuble, caractérisé en ce qu'on réalise les étapes suivantes :
a) stocker des données basées sur des mesures issues d'un auto-réfractomètre ;
b) entrer des données complémentaires relatives à la lentille de contact autres que celles fournies par l'auto-réfractomètre, à savoir des données relatives à la forme de la lentille, la nature du matériau formant la lentille et la fréquence de port de la lentille ;
c) déterminer les caractéristiques d'une lentille de contact disponible chez le fournisseur sur la base d'une part, des données de mesure issues de l'auto-réfractomètre qui ont été préalablement stockées et d'autre part, des données complémentaires préalablement entrées par l'utilisateur à l'étape b);
d) identifier l'emplacement, au sein du meuble, de cette lentille de contact ;
e) sortir du meuble le boîtier comportant cette lentille de contact.

Le procédé selon l'invention pourra également comprendre l'une au moins des caractéristiques suivantes, prises seules ou en combinaison :
- il comprend, en outre, les étapes suivantes :
   f) au moins réaliser une mesure de surréfraction sur un patient muni de la lentille de contact dont les caractéristiques ont été déterminées à l'étape c) ;
   g) déterminer les caractéristiques d'une nouvelle lentille de contact sur la base d'une part, des données de mesures réalisée à l'étape f) et d'autre part, des caractéristiques de la lentille de contact déterminées à l'étape c) ;
   h) identifier l'emplacement, au sein du meuble (1), de cette nouvelle lentille de contact ;
   i) sortir du meuble (1) le boîtier comportant cette nouvelle lentille de contact.
- la lentille de contact dont les caractéristiques ont été déterminées l'étape c) étant une lentille sphérique, l'étape g) consiste à déterminer la sphère, le cylindre et l'axe de la nouvelle lentille de contact ;
- la lentille de contact dont les caractéristiques ont été déterminées l'étape c) étant une lentille torique, l'étape f) consiste à réaliser au moins une mesure de surréfraction et une mesure de rotation et l'étape g) consiste à déterminer la sphère, le cylindre et l'axe de la nouvelle lentille de contact ;
- la lentille de contact dont les caractéristiques ont été déterminées l'étape c) étant une lentille progressive, l'étape f) consiste à réaliser des mesures de surréfraction, duochrome, d'acuité visuelle en vision de près et de loin, à déterminer l'oeil adelphe ou dominant du patient et à fournir l'âge du patient et l'étape g) consiste à déterminer la sphère et/ou l'addition de la nouvelle lentille de contact ;
- une étape pour alerter l'utilisateur de l'emplacement de la lentille de contact au sein du meuble ;
- une étape consistant à distribuer automatiquement le boîtier comportant la lentille de contact ;
- une étape dans laquelle on identifie des informations contenues sur le boîtier de cette lentille de contact, ledit boîtier étant muni d'un code-barres ou d'une puce RFID ;
- une étape dans laquelle on transmet les informations identifiées sur le boîtier de la lentille de contact à distance, par exemple par l'intermédiaire du réseau Internet, afin d'assurer le réassortiment des lentilles de contact ;
- une étape dans laquelle on initie le réassortiment des lentilles de contact lorsque le nombre de boîtiers comportant des lentilles de contact identifié et transmis à distance est en-dessous d'une valeur seuil prédéfinie ;
- une étape dans laquelle on transmet les données caractéristiques de la lentille dont les informations présentes sur le boîtier ont été identifiées vers l'ordinateur personnel de l'utilisateur.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 représente un dispositif conforme à l'invention ;
- la figure 2 est une représentation schématique fonctionnelle du dispositif représenté sur la figure 1, avec son environnement ;
- la figure 3 est une autre représentation schématique fonctionnelle du dispositif représenté sur la figure 1.

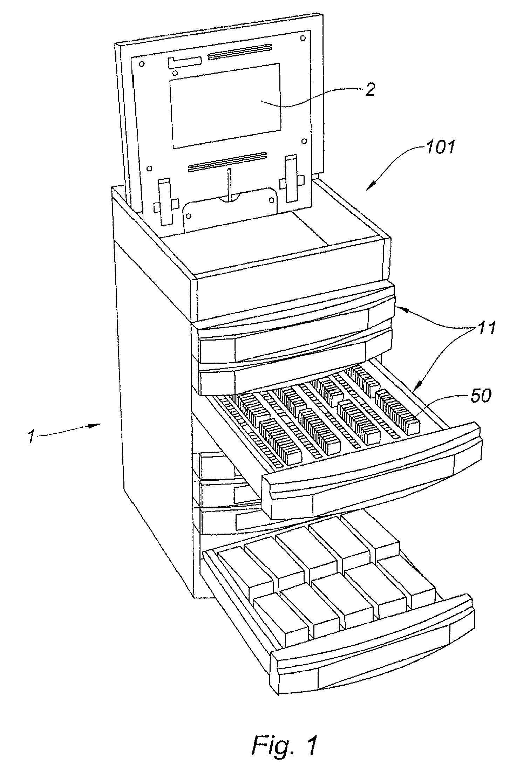

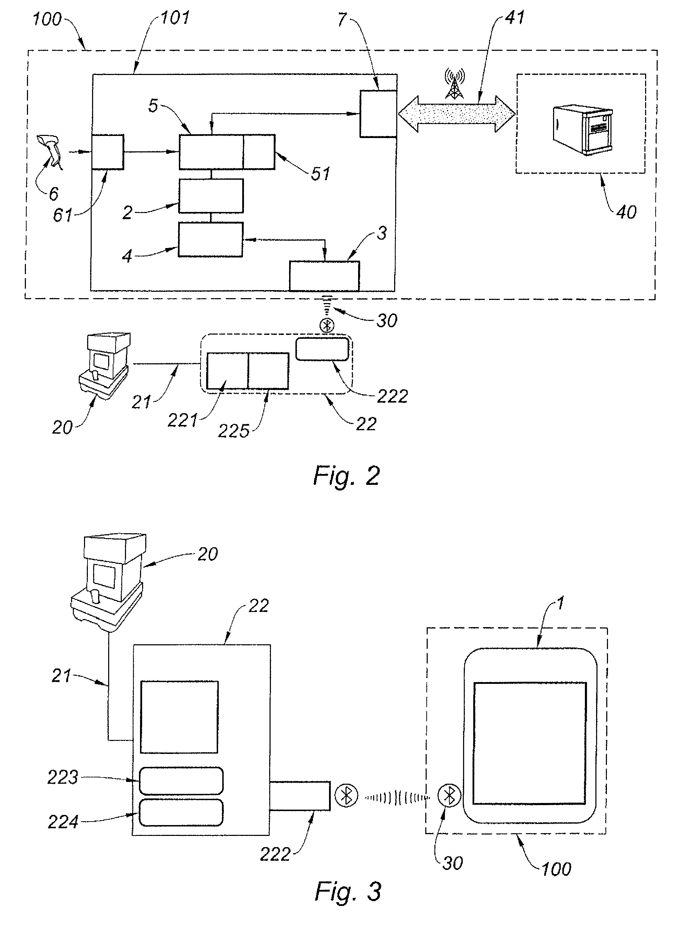

Un exemple de dispositif de sélection et de réassortiment de lentilles de contact conforme à l'invention est représenté sur la figure 1 et la figure 2.

Le dispositif 100 comporte une partie 101 installée localement chez l'ophtalmologiste et une partie 40 distante, installée chez le fournisseur.

La partie 101 se présente sous la forme d'un meuble 1 agencé pour recevoir une pluralité de lentilles de contact 50 et logeant plusieurs fonctionnalités décrites ci-après.

L'auto-réfractomètre 20 est généralement relié par câble 21 à l'ordinateur personnel 22 de l'ophtalmologiste. Cet ordinateur personnel 22 comprend un logiciel 221 de traitement des données issues de l'auto-réfractomètre 20 et une mémoire 225 pour stocker les données ainsi traitées.

Pour assurer la communication entre l'ordinateur personnel 22 de l'ophtalmologiste et le dispositif conforme à l'invention, un module 222 de gestion de liaison 30 Bluetooth® de cet ordinateur 22 peut être mis à profit. Dans ce cas, le dispositif 100 comprend également un module de gestion de liaison 3 Bluetooth®, servant à la communication avec le logiciel 221 de traitement des données issues de l'auto-réfractomètre 20 et/ou la mémoire 225.

Les données issues de ce logiciel 221 peuvent donc être transférées dans le dispositif 100 conforme à l'invention et stockées.

A cet effet, le dispositif 100 comprend un moyen 4 pour stocker (mémoire) les données de mesures issues de l'auto-réfractomètre 20. Ce moyen de stockage 4 est avantageusement une mémoire « flash » stockant les données de façon temporaire pour leur traitement ultérieur.

La liaison Bluetooth® n'est qu'un exemple et d'autres modes de communication entre l'ordinateur personnel 22 de l'ophtalmologiste et le dispositif 100 conforme à l'invention peuvent être envisagés comme une liaison Wifi ou une liaison câblée sur réseau local.

Il pourrait même être envisagé de ne prévoir aucun mode de communication entre l'ordinateur personnel 22 de l'ophtalmologiste et le dispositif 100 selon l'invention. Dans ce cas, le moyen d'affichage 2 du dispositif peut prévoir l'affichage d'un clavier numérique permettant à l'ophtalmologiste de rentrer directement les données issues de l'auto-réfractomètre. En variante, le dispositif 100 pourrait prévoir un clavier à cet effet.

Dans tous les cas, le moyen d'affichage 2 sert à afficher des données relatives à la lentille. En particulier, ce moyen d'affichage 2 permet d'afficher une fenêtre dans laquelle des informations relatives à la sélection de la lentille de contact que l'ophtalmologiste cherche justement à sélectionner sont proposées, informations autres que celles fournies par l'auto-réfractomètre 20.

Ces informations portent notamment sur la fréquence de port de la lentille de contact (journalière, mensuelle), la nature du matériau de la lentille de contact et la forme de cette lentille (sphérique, torique, ...).

Le dispositif 100 comprend également un moyen de traitement de données 5, tel qu'un processeur ou un ensemble de processeurs, qui associé à un module de calcul 51, permet de déterminer la lentille de contact qui est la plus appropriée pour son patient.

A cet effet, le module de calcul 51 s'appuie sur les mesures issues de l'auto-réfractomètre 20 (sphère, cylindre, axe, addition, rayon(s) et éventuellement mais pas nécessairement sur l'axe associé au ou à chaque rayon) qui ont été préalablement stockées dans la mémoire 4. Il s'appuie également sur les informations affichées sur le moyen d'affichage 2 (forme de la lentille de contact, nature du matériau formant la lentille, fréquence de port de cette lentille), demandées à l'ophtalmologiste et qui ont été préalablement entrées par l'ophtalmologiste dans le dispositif 100.

Dans une version basique, le module de calcul 51 a donc en entrée les données suivantes :
- forme de la lentille de contact (lié au type d'amétropie), nature du matériau formant la lentille, fréquence de port de cette lentille ; entrées par l'ophtalmologiste lui-même dans le dispositif 100 ;

Sphère, cylindre, axe, addition et deux mesures de rayons de courbure de la cornée (kératométrie max et min) ; issues de l'auto-réfractomètre 20 et éventuellement corrigées subjectivement par l'ophtalmologiste.

Avec les données d'entrées précisées ci-dessus, le module de calcul 51 détermine la lentille de contact la mieux adaptée au défaut visuel du patient avec les critères suivants :
1) forme de la lentille (amétropie) : correspondance exacte ;
2) matériau de la lentille : correspondance exacte ;
3) fréquence de port de la lentille : correspondance exacte ;
4) rayon : identification des rayons disponibles chez le fournisseur.

Les valeurs extrémales de rayon disponibles chez le fournisseur pour des lentilles de contact « standard » permettent de définir une gamme [Rₘᵢₙ ; Rₘₐₓ].

Cette gamme [Rₘᵢₙ ; Rₘₐₓ] dépend de la grille du fournisseur et peut évoluer dans le temps pour chaque fournisseur.

Le rayon doit alors être compris entre les valeurs minimale (Rₘᵢₙ) et maximale (Rₘₐₓ) de ce rayon qui sont disponibles dans la grille de lentilles « standard » proposées par le fournisseur.

Dans l'hypothèse (rare) où une seule mesure de ce rayon de courbure est réalisée par l'ophtalmologiste, on peut sélectionner directement cette mesure, qui peut être soit objective soit subjective comme expliqué précédemment.

Dans le cas plus général où deux mesures du rayon de courbure de la cornée (kératométrie max ; kératométrie min) sont réalisées par l'ophtalmologiste, ce rayon est déterminé en fonction de ces deux mesures du rayon de courbure de la cornée et, éventuellement de la sphère, issues de l'auto-réfractomètre 20.

Par exemple, on peut déterminer le rayon en réalisant une moyenne arithmétique entre les deux mesures de rayons réalisées avec l'auto-réfractomètre. Puis, on vérifie que ce rayon est dans la gamme [Rₘᵢₙ ; Rₘₐₓ]. Dans l'affirmative, on trouvera une lentille adéquat.
5) sphère : identification d'une valeur de sphère disponible chez le fournisseur

Les valeurs extrémales de sphère disponibles chez le fournisseur pour des lentilles de contact « standard » permettent de définir une gamme [SphereMin ; SphereMax].

Si la valeur de sphère se situe dans cette gamme et correspond à une valeur exacte disponible dans la grille du fournisseur, alors on sélectionne cette lentille (cas trivial).

Si la valeur de la sphère se situe dans la gamme [SphereMin ; SphereMax], mais que la valeur exacte de cette sphère, issue de l'auto-réfractomètre sous forme objective ou subjective, n'est pas identifiée parmi les lentilles de contact « standard » disponibles chez le fournisseur, alors :
- si la valeur de sphère (issue de la mesure de l'auto-réfractomètre) est négative, on sélectionne une lentille de contact disponible dans la grille du fournisseur en fonction de la plus petite valeur, en valeur absolue, de cette sphère ;
- si la valeur de sphère (issue de la mesure de l'auto-réfractomètre) est positive, on sélectionne une lentille de contact disponible dans la grille du fournisseur en fonction de la plus grande valeur, en valeur absolue, de cette sphère.

6) cylindre : identification d'une valeur de cylindre disponible chez le fournisseur

Les valeurs extrémales de sphère disponibles chez le fournisseur pour des lentilles de contact « standard » permettent de définir une gamme [CylMin; CylMax].

Si la valeur du cylindre se situe dans cette gamme et correspond à une valeur exacte disponible dans la grille du fournisseur, alors on sélectionne cette lentille (cas trivial).

Si la valeur de cylindre se situe dans cette gamme, mais que la valeur exacte n'est pas identifiée parmi les lentilles disponibles dans la grille du fournisseur, alors on sélectionne une lentille de contact en fonction de la plus petite valeur, en valeur absolue, de ce cylindre.
7) axe (astigmatisme) : identification d'une valeur d'axe disponible chez le fournisseur

Les valeurs extrémales de sphère disponibles chez le fournisseur pour des lentilles de contact « standard » permettent de définir une gamme [AxeMin ; AxeMax].

Si la valeur d'axe se situe dans cette gamme et correspond à une valeur exacte disponible dans la grille du fournisseur, alors on sélectionne cette lentille (cas trivial).

Si la valeur d'axe se situe dans cette gamme, mais que la valeur exacte n'est pas identifiée parmi les lentilles de contact disponibles dans le meuble, alors :
Si 0 < axe < 45°, on sélectionne une lentille de contact disponible dans la grille du fournisseur présentant la valeur d'axe la plus proche de la valeur nulle.
Si 45° ≤ axe < 135°, on sélectionne une lentille de contact présentant la valeur d'axe la plus proche de 90°.
Si 135° ≤ axe < 180°, on sélectionne la lentille de contact présentant la valeur d'axe la plus proche de 180°.
8) addition : plusieurs cas sont à distinguer.

Si la lentille de contact présente un profil d'addition unique, alors on sélectionne ce profil d'addition (cas trivial).

Si la lentille de contact présente deux profils d'addition (profil bas = première valeur d'addition, profil haut = deuxième valeur d'addition, supérieure à la première valeur d'addition) :
- soit l'addition est inférieure ou égale à une valeur seuil prédéfinie (par exemple valeur seuil = 2.25 dioptries) et dans ce cas, on sélectionne le profil bas.
- soit l'addition est strictement supérieure à cette valeur seuil et dans ce cas, on sélectionne le profil haut.

Si la lentille de contact présente plus de deux profils d'addition, alors :
- si l'adition est strictement inférieure à la valeur seuil (par exemple valeur seuil = 2.25 dioptries), alors on sélectionne la lentille de contact répondant à ce critère ;
- si l'addition vaut la valeur seuil ou plus, alors on ajoute une addition de valeur prédéterminée à la sphère obtenue pour sélectionner la lentille de contact (dans le cas d'une addition de valeur 2.25, alors la valeur prédéterminée à ajouter à la sphère est de 0.25 ; dans le cas d'une addition de valeur 3, alors la valeur prédéterminée à ajouter à la sphère est de 0.5)

La détermination de la lentille de contact (lentille d'essai) la plus appropriée s'effectue avantageusement en respectant l'ordre 1) à 8) ci-dessus. Ainsi, une fois la condition 1) respectée, on chercher une lentille de contact disponible dans la grille du fournisseur capable de répondre à la condition 2) et ainsi de suite pour l'ensemble des conditions. Un autre ordre pourrait cependant être envisagé dans la sélection de la lentille de contact.

Ceci est particulièrement intéressant car cela évite à l'ophtalmologiste de faire appel à son expérience ou, dans certains cas, à des tableaux d'équivalence, bien connus de l'homme du métier, entre des données relatives aux lentilles correctives pour lunettes et les données correspondantes pour les lentilles de contact.

Le traitement des données est donc plus rapide et moins sujet à d'éventuelles erreurs d'appréciations de l'ophtalmologiste.

Par ailleurs, le moyen de traitement 5 associé au module de calcul 51, après avoir permis la détermination de la lentille de contact la plus appropriée pour son patient, permet d'identifier l'emplacement, au sein du meuble 1, de cette lentille de contact.

Le traitement réalisé permet ainsi non seulement de sélectionner les caractéristiques de la lentille de contact qui est la plus appropriée pour le patient, mais permet également d'identifier dans quel tiroir 11 du meuble 1 se situe cette lentille.

Le moyen d'affichage 2 peut alors être utilisé pour afficher la position de la lentille dans le meuble. En variante à la seule alerte visuelle consistant à afficher l'emplacement de la lentille recherchée sur le moyen d'affichage, on peut envisager une alerte sonore et/ou une alerte visuelle.

Si la lentille de contact susceptible d'être la plus appropriée pour le patient ne répond pas à l'un des critères définis par les références 1) à 8) ci-dessus, alors cela signifie que la lentille de contact n'existe pas dans la grille de lentilles de contact « standard » proposée par le fournisseur. Dans un tel cas, l'ophtalmologiste doit alors passer commande d'une lentille sur mesure (lentille de contact RX) auprès de son fournisseur.

Par ailleurs, il est possible qu'après avoir déterminé, avec le module de calcul 51, la lentille de contact la plus appropriée au patient, celle-ci soit bien présente dans la gamme de lentilles de contact « standard » proposée par le fournisseur, mais que le meuble 1 n'ait pas été agencé pour prévoir cette lentille de contact. En effet, pour des raisons d'efficacité, le meuble 1 comporte de préférence les lentilles les plus prescrites. Dans ce cas, l'ophtalmologiste doit également passer commande de cette lentille de contact « standard » auprès de son fournisseur.

Cependant, il convient de rappeler que, dans un mode de réalisation particulièrement avantageux, l'invention vise également que des lentilles de contact « standard », pour lesquelles un emplacement est prévu initialement dans le meuble 1, ne soient pas en rupture.

A cet effet, le dispositif 100 peut comprendre un moyen 6 pour identifier le boîtier comportant la lentille de contact préalablement sélectionnée et identifiée. Ce moyen 6 peut être un lecteur de code-barres ou un lecteur fonctionnant par radio-identification (RFID pour « Radio Frequency Identification » selon la terminologie anglo-saxonne). A cet effet, on met à profit un code-barres ou, selon le cas, une puce RFID, présent(e) sur chaque boîtier de lentilles de contact.

Sur la figure 2, on a représenté un lecteur de code-barres 6, manipulable par l'ophtalmologiste.

Dans le cas d'un lecteur de fonctionnant par radio-identification, il est envisageable d'insérer le lecteur directement sur le meuble 1. L'ophtalmologiste doit alors passer le boîtier de la lentille de contact, muni d'une puce RFID, devant le lecteur RFID. Avantageusement, chaque tiroir 11 du meuble 1 pourra être muni d'un lecteur de ce type, si bien qu'une identification automatique d'un boîtier de lentilles de contact peut être réalisée à chaque sortie du tiroir 11 concerné. Une fois ce boîtier identifié, un module 61 appartenant également au dispositif 100, relié au moyen d'identification 6, permet de prendre en compte les informations comprises dans le code-barres.

La prise en compte des informations identifiées sur le boîtier permet au moyen de traitement de données 5 du dispositif de réaliser plusieurs actions particulièrement avantageuses.

En premier lieu, cela permet de transmettre, par l'intermédiaire d'un module 7 et une liaison à distance 41, telle que le réseau Internet, les données identifiées sur le boîtier de lentilles vers un serveur informatique 40 distant logé chez le fournisseur de lentilles de contact.

De ce fait, le fournisseur de lentilles de contact sait quelle lentille a été utilisée par l'ophtalmologiste. Cela permet une gestion active du réassort des différentes lentilles de contact restantes dans le meuble 1 présente dans le cabinet de l'ophtalmologiste. En effet, le fournisseur peut alors identifier les lentilles de contact qui risquent de manquer et réaliser une livraison ciblée. Cela évite à l'ophtalmologiste de passer commande, d'où un gain de temps. Cela permet aussi d'éviter d'éventuelles erreurs que pouvaient commettre l'ophtalmologiste lors d'une commande.

En second lieu, le dispositif 100 peut envoyer les données reçues par le module 61 en direction de l'ordinateur personnel 22 de l'ophtalmologiste, par l'intermédiaire de la liaison 30 précitée. Un fichier 224 prenant en compte l'ensemble des caractéristiques de la lentille de contact proposée par l'ophtalmologiste est ainsi stocké avec le fichier 223 initial relatif à son patient. Un suivi de l'évolution des types de lentilles de contact successivement proposées au patient peut donc être réalisé.

Sur ce dernier aspect, on pourra se référer à la figure 3.

Enfin, le fait de scanner le boîtier de la lentille confère une traçabilité permettant d'identifier aisément un lot de lentilles de contact défectueuses.

Si l'ophtalmologiste souhaite remettre en place le boîtier de lentille de contact sortie du meuble, il pourra à nouveau identifier ce boîtier avec le moyen 6 pour éviter que le stock ne soit décrémenté.

Il convient de noter que les données issues de l'auto-réfractomètre 20, traitées par le logiciel 221 et stockées dans la mémoire 225 de l'ordinateur personnel 22 de l'ophtalmologiste peuvent être des données objectives de la mesure effectivement réalisée par l'auto-réfractomètre. Elles peuvent également être des données subjectives, c'est-à-dire des données modifiées par l'ophtalmologiste lui-même, sur la base des données objectives fournies par l'auto-réfractomètre 20. Dans tous les cas, les données objectives ou subjectives sont établies au niveau de l'ordinateur personnel 22 de l'ophtalmologiste, à l'aide du logiciel.

Cet aspect ne change rien au fonctionnement du dispositif 100 conforme à l'invention, lequel reçoit donc indifféremment des données objectives ou subjectives, qui sont dans chaque cas basées sur les données de mesure de l'auto-réfractomètre 20.

Il convient également de noter que, de façon avantageuse, le module de calcul 51 est implanté au sein même du dispositif 100, à savoir chez l'ophtalmologiste. En variante, on pourrait cependant envisager d'implanter ce module de calcul 51 au niveau du serveur distant 40. Toutefois, cette variante est moins avantageuse car tributaire du bon fonctionnement de la liaison distante 41.

De plus, la description faite précédemment part du principe que l'ophtalmologiste entre les données relatives à la forme de la lentille de contact (lié à l'amétropie), à la nature du matériau formant la lentille et à la fréquence de port par l'intermédiaire du moyen d'affichage 2 présent dans le dispositif 100.

Or, dans certains cas, l'ordinateur personnel 22 de l'ophtalmologiste prévoit une interface utilisateur lui proposant d'entrer ces informations. Dans ce cas, le dispositif 100, par l'intermédiaire du moyen de traitement 5 et du module da calcul 51, peut initier le calcul de la lentille de contact la plus appropriée pour le patient directement sur la base de l'ensemble des données transmises entre l'ordinateur personnel 22 de l'ophtalmologiste et le dispositif 100. Dans un tel cas, le moyen d'affichage 2 n'est pas nécessaire.

Par ailleurs, le meuble 1 décrit précédemment à l'appui des figures annexées est un meuble 1 à tiroirs 11.

En variante (non représentée sur les figures annexées), on pourrait envisager un meuble 1 se présentant sous la forme d'un distributeur automatique de boîtiers de lentilles de contact. Le fonctionnement d'un dispositif conforme à l'invention comprenant un tel meuble, ci-après nommé meuble distributeur, est similaire à ce qui a été décrit précédemment, dans ses différentes variantes, sur le transfert des données entre l'ordinateur personnel 2 de l'ophtalmologiste et le dispositif 100, sur le stockage dans la mémoire 4, sur la sélection des lentilles de contacts par l'intermédiaire du moyen de traitement 5 et du module de calcul 51 associé.

En revanche, avec un tel meuble distributeur, les moyens pour identifier les informations contenues sur le boîtier d'une lentille de contact diffèrent.

De plus, le meuble distributeur comporte alors des moyens pour prélever automatiquement le boîtier correspondant à la lentille sélectionnée par le module de calcul 51. Pour ce faire, les lentilles de contact sont agencées dans le meuble distributeur de façon analogue à leur agencement dans le meuble 1 à tiroirs.

Chaque étage du meuble distributeur peut donc être assimilé à un tiroir 11 du meuble 1 représenté sur la figure 2. En d'autres termes, le meuble distributeur peut être agencé pour recevoir, à chaque étage, plusieurs séries de lentilles de contact d'un même modèle pour lequel chaque série de lentilles de contact se distingue d'une autre série de lentilles de contact par la puissance de la correction ophtalmique. D'un étage à l'autre, on peut prévoir différents modèles de lentilles.

Un moyen de prélèvement du boîtier de lentilles de contact est alors prévu. Il peut par exemple s'agir d'une gâchette électrique capable d'extraire le boîtier concerné de son emplacement, auquel est associée une gouttière capable de transférer le boîtier sorti de son emplacement pour l'acheminer vers la sortie du meuble distributeur.

Le moyen (premier lecteur) pour identifier les informations contenues sur le boîtier (lecteur fonctionnant par RFID ou code-barres, par exemple) est alors avantageusement implanté à l'intérieur du meuble distributeur, de préférence au niveau de la sortie de ce meuble par laquelle l'ophtalmologiste peut récupérer ledit boîtier. En variante, ce lecteur peut être disposé à l'intérieur du meuble, mais non à la sortie, ou sur la partie externe du meuble ou encore à l'extérieur du meuble, comme représenté sur la figure 2.

L'éventuel transfert des informations récupérées par le lecteur au serveur distant 40 s'effectue alors comme décrit précédemment pour le dispositif 100 décrit à l'appui des figures 1 à 3, tout comme l'éventuel transfert de ces informations en direction de l'ordinateur personnel 2 de l'ophtalmologiste.

Si l'ophtalmologiste souhaite remettre le boîtier sorti du meuble distributeur (par exemple s'il a effectué une correction subjective des données objectives issues de l'auto-réfractomètre qui lui semble erroné), il peut l'insérer dans une entrée prévue à cet effet dans le meuble, telle qu'une fente.

Un moyen (deuxième lecteur) pour identifier les informations contenues par exemple sur une puce RFID ou un code-barres présente sur le boîtier est avantageusement installé à l'intérieur du meuble au niveau de cette entrée, pour l'identifier et s'assurer ainsi que cette lentille de contact n'a pas été décrémentée du meuble. En variante, ce deuxième lecteur peut être disposé à l'intérieur du meuble, mais non à l'entrée, ou sur la partie externe du meuble ou encore à l'extérieur du meuble, comme représenté sur la figure 2.

Enfin, lorsqu'une entrée est prévue sur le meuble distributeur, ce dernier comprend des moyens pour remettre le boîtier à sa place.

Les différents moyens du meuble distributeur relatifs à la remise en place éventuelle du boîtier de lentilles de contact préalablement sortis sont facultatifs, mais serons avantageusement prévus.

L'invention n'est pas limitée au seul dispositif 100 décrit précédemment dans ses différentes variantes.

Ainsi, le module 51 peut prévoir d'autres fonctions visant à proposer une nouvelle lentille d'essai, après que le patient a testé, pendant quelques heures ou quelques semaines la lentille d'essai prescrite par l'ophtalmologiste. Il s'agit donc d'une étape de contrôle, qui peut permettre de proposer une nouvelle lentille de contact.

### Cas n°1 : contrôle d'une lentille sphérique.

L'ophtalmologiste choisi l'essai ou l'un des essais effectués précédemment, qui ont été stockés dans la mémoire 4. La liste de ou de chaque essai réalisé pour un patient sera affiché sur le moyen d'affichage 2, avec une sélection par date et nom de la lentille correspondante. En général, l'ophtalmologiste choisira le dernier essai, lequel correspond à la lentille d'essai que porte le patient. Toutes les données caractéristiques de cette lentille sont donc connues.

De manière connue, l'ophtalmologiste renseigne alors des valeurs correspondant à la biomicroscopie de l'oeil pour indiquer si la lentille a des paramètres d'adaptation convenables en centrage, mobilité et recouvrement. En cas de mauvais centrage ou de mauvais recouvrement, par exemple, l'ophtalmologiste pourra demander à être rappelé par le pole technique du fournisseur.

Dans la plupart des cas, il n'y aura de difficulté avec le positionnement de la lentille.

Alors, l'ophtalmologiste renseigne alors les paramètres correspondant à la surréfraction monoculaire, pour chacun des deux yeux muni d'une lentille d'essai. Pour obtenir ces données, il réalise des mesures tout à fait classiques, le patient portant les lentilles d'essais. En effet, la surréfraction est la réfraction obtenue sur une lentille déjà portée.

Il entre alors les données de surréfraction mesurées dans le dispositif 100 conforme à l'invention, par exemple par l'intermédiaire de son ordinateur personnel 22.

Avec ces données d'entrées précisées ci-dessus, le module de calcul 51, associé au moyen de traitement 5, détermine une nouvelle lentille de contact d'essai mieux adaptée de la manière suivante:
- sphère = somme de la sphère de la lentille d'essai choisie par l'ohptalmologiste (par exemple, celle que porte le patient) et de la sphère de surréfraction ;
- cylindre = cylindre de surréfraction ;
- axe (astigmatisme) = axe de surréfraction ;

Il convient alors de noter que la lentille obtenue n'est pas forcément une lentille sphérique.

Une fois le calcul effectué par le module 51, l'identification et la sélection de la nouvelle lentille d'essai s'effectue comme décrit précédemment. En particulier, le module de traitement 5 détermine si la nouvelle lentille d'essai est disponible dans le meuble ou doit être commandée.

En définitive, lorsque la lentille préalablement identifiée est une lentille sphérique, le moyen 5 de traitement de données associé au module de calcul 51 est agencé pour déterminer la sphère, le cylindre et l'axe de la nouvelle lentille à partir des caractéristiques de la lentille préalablement identifiée et d'une mesure de surréfraction réalisée sur le patient muni de cette lentille préalablement identifiée.

### Cas n°2 : contrôle d'une lentille torique.

L'ophtalmologiste choisi l'essai ou l'un des essais effectués précédemment, qui ont été stockés dans la mémoire 4. En général, l'ophtalmologiste choisira le dernier essai, lequel correspond à la lentille d'essai que porte le patient. Toutes les données caractéristiques de cette lentille sont donc connues.

De manière connue, l'ophtalmologiste renseigne alors des valeurs correspondant à la biomicroscopie de l'oeil pour indiquer si la lentille a des paramètres d'adaptation convenables en centrage, mobilité et recouvrement. En cas de difficulté avec le positionnement de la lentille, il pourra appeler le pole technique du fournisseur.

Dans la plupart des cas, il n'y aura de difficulté avec le positionnement de la lentille.

L'ophtalmologiste réalise alors une mesure, selon une méthode connue, de la rotation de chaque lentille.

L'ophtalmologiste doit alors déterminer les paramètres correspondant à la surréfraction monoculaire, pour chacun des deux yeux muni d'une lentille d'essai.

L'ophtalmologiste entre alors, dans le dispositif 100, les mesures de rotation et les mesures liées à la surréfraction monoculaire.

Avec ces données d'entrées précisées ci-dessus, le module de calcul 51, avec le moyen de traitement 5, peut alors déterminer une nouvelle lentille de contact d'essai mieux adaptée de la manière suivante en déterminant les paramètres sphère, cylindre et axe, qui font intervenir la mesure de rotation.
- sphère = B3 + B6 + (B4+B7)/2 - B13/2;
- cylindre = B13;
- axe : SI B14 ≤0,5 ALORS axe = ENT[B14+180+ 0,499] ; SINON axe = ENT[B14+0,499].

Avec :
ENT = opérateur mathématique désignant la partie entière d'un nombre,
B3 = sphère de la lentille d'essai choisie (par exemple, celle que porte le patient en venant au contrôle),
B4 = cylindre de la lentille d'essai choisie,
B5 = axe de la lentille d'essai choisie,
B6 = sphère de surréfraction,
B7 = cylindre de surréfraction,
B8 = axe de surréfraction,
B9 = mesure de rotation de la lentille d'essai choisie.

Et :
B11 = B4*COS((B5-B9)*P)/90)+B7*COS(B8*PI/90),
B12 = B4*S)N((B5-B9)*P)/90)+B7*SIN(B8*P)/90),
B13 = - ((B11^2+B12^2)^0,5),

### Calcul de B14:

```
 Si B12=0
 Alors
      Si B13-11=0
             Alors B14 = 180 - B9
      Sinon B14 = 90 -B9
      Fin Si
      Sinon
      B14 = B9 + ATAN((B13-B11)/B12)*180/PI
      Fin Si
```

où: PI vaut approximativement 3,14159.

Une fois le calcul effectué par le module 51, l'identification et la sélection de la nouvelle lentille d'essai s'effectue comme décrit précédemment. En particulier, le module de traitement 5 détermine si la nouvelle lentille d'essai est disponible dans le meuble ou doit être commandée.

En définitive, lorsque la lentille préalablement identifiée est une lentille torique, le moyen 5 de traitement de données associé au module de calcul 51 est agencé pour déterminer la sphère, le cylindre et l'axe de la nouvelle lentille à partir des caractéristiques de la lentille préalablement identifiée et de mesures de surréfraction et de rotation réalisées par l'utilisateur sur le patient muni de cette lentille préalablement identifiée.

### Cas n°3 : contrôle d'une lentille progressive.

L'ophtalmologiste choisi l'essai ou l'un des essais effectués précédemment, qui ont été stockés dans la mémoire 4. En général, l'ophtalmologiste choisira le dernier essai, lequel correspond à la lentille d'essai que porte le patient. Toutes les données caractéristiques de cette lentille sont donc connues.

De manière connue, l'ophtalmologiste renseigne alors des valeurs correspondant à la biomicroscopie de l'oeil pour indiquer si la lentille a des paramètres d'adaptation convenables en centrage, mobilité et recouvrement. En cas de difficulté avec le positionnement de la lentille, il pourra appeler le pole technique du fournisseur.

Dans la plupart des cas, il n'y aura de difficulté avec le positionnement de la lentille.

L'ophtalmologiste indique alors dans le dispositif 100 l'oeil dominant du patient, droit ou gauche. Par définition, l'oeil non dominant est l'oeil adelphe. Plus précisément, l'oeil adelphe ou oeil le plus convexe est l'oeil pour lequel la sphère de la lentille d'essai choisie, pour déterminer la nouvelle lentille d'essai, est maximale en valeur algébrique.

L'ophtalmologiste renseigne également dans le dispositif 100, l'âge du patient.

Puis, l'ophtalmologiste indique dans le dispositif 100 la préférence du patient sur le test duochrome rouge/vert en vision de loin : rouge, vert ou équilibré. La valeur « vert » fait apparaître une fenêtre d'alerte informant l'ophtalmologiste qu'il devra modifier la correction afin de tendre vers le rouge.

L'ophtalmologiste doit alors renseigner, dans le dispositif 100, des valeurs d'acuité visuelle binoculaire, le patient portant la lentille d'essai choisie:
- acuité visuelle en vision de loin (AVVL): une liste déroulante où les valeurs sont libellées selon l'échelle d'acuité visuelle de Monoyer est affichée. Dans la liste déroulante, la taille de police utilisée augmente selon que l'acuité baisse afin de faciliter la lisibilité de l'échelle,
- acuité visuelle en vision de près (AVVP): une liste déroulante où les valeurs sont libellées selon l'échelle d'acuité visuelle de Parinaud est affichée. Dans la liste déroulante, la taille de police utilisée augmente selon que l'acuité baisse afin de faciliter la lisibilité de l'échelle. Il doit aussi préciser la distance à laquelle il effectue ce test.

Le module de calcul 51 opère alors de la façon suivante, à partir des données entrées par l'ophtalmologiste dans le dispositif 100, par exemple à l'aide de son ordinateur personnel 22.

Le module de calcul 51 se base sur la définition d'un score réalisé à partir des échelles de Monoyer (AWL) et de Parainaud (AVVP), de la façon suivante :

### Pour l'AVVL :

12/10 = 8 points
11/10=7 points
10/10 = 6 points
9/10 = 5 points
8/10 = 4 points
7/10 = 3 points
6/10 = 2 points
5/10 = 1 point

### Pour l'AVVP :

P2 = 8 points
P3 = 6 points
P4 = 4 points
P5 = 2 points.

Le score est alors défini par la somme des points obtenus pour l'AVVL et l'AVVP.

Si score ≥ 12 alors, on utilise l'algorithme de niveau 1 suivant, implémenté par le module de calcul 51 :
- Si AVVL ≥ 10/10, alors on augmente de la sphère de l'oeil adelphe de +0.25 dioptrie ;
- Si AVVL ≤ 9/10 et AVVP ≥ P3, alors on diminue la sphère de l'oeil dominant de -0.25 dioptrie,
- Si les deux yeux sont en LOW et 52ans ≤ âge du patient ≤ 55ans, alors on augmente l'addition de l'oeil adelphe de BAS (« LOW » selon la terminologie anglo-saxonne, bien connue de l'homme du métier) à HAUT (« HIGH » selon la terminologie anglo-saxonne),
- Si l'oeil adelphe est déjà en LOW et âge du patient > 55ans et AVVL ≥ 10/10, alors on augmente l'addition de l'oeil dominant de LOW à HIGH,
- Si aucune des quatre conditions ci-dessus n'est respectée, alors on utilise l'algorithme du niveau 3.

Si score 9 < score < 12 alors, on utilise l'algorithme de niveau 2 suivant:
- Si AWL > 10/10 et duo-chrome sur vert et/ou surréfraction convexe ou plan, alors on augmente la sphère des deux lentilles d'essais choisies de +0.25 dioptrie,
- Si AVVL ≤ 8/10 et AWP > P2, alors on diminue la sphère des deux lentilles d'essais choisies de -0.25 dioptrie,
- Si aucune des deux conditions ci-dessus n'est respectée, alors on utilise l'algorithme de niveau 3.

Enfin, si score ≤ 9 ou que les algorithmes de niveau 1 et 2 n'ont pas abouti (pas de solution), alors on utilise l'algorithme niveau 3 suivant :
Si l'âge du patient est compris entre 45ans et 48ans (inclus), alors on diminue la sphère (surréfraction) des deux lentilles d'essais de -0.25 dioptrie,
Si l'âge du patient est compris entre 48ans (exclu) et 50ans (inclus), alors on diminue la sphère de -0.25 dioptrie sur la lentille d'essai de l'oeil dominant et de -0.50 dioptrie sur la lentille d'essai de l'oeil adelphe,
Si l'âge du patient est compris entre 50ans (exclu) et 52ans (inclus), alors on diminue la sphère des deux lentilles d'essais de -0.25 dioptrie,
Si l'âge du patient est compris entre 52ans (exclu) et 55ans (inclus), alors on diminue la sphère de -0.50 dioptrie add LOW sur l'oeil dominant et de -0.75 dioptrie add HIGH sur l'oeil adelphe,
Si l'âge du patient est au-delà de 55ans, alors on diminue la sphère de -0.75 dioptrie sur les deux lentilles, en add LOW sur l'oeil dominant et en add HIGH sur l'oeil adelphe.

Une fois le calcul effectué par le module 51, l'identification et la sélection de la nouvelle lentille d'essai s'effectue comme décrit précédemment. En particulier, le module de traitement 5 détermine si la nouvelle lentille d'essai est disponible dans le meuble ou doit être commandée.

En définitive, lorsque la lentille préalablement identifiée est une lentille progressive, le moyen 5 de traitement de données associé au module de calcul 51 est agencé pour déterminer la sphère et/ou l'addition de la nouvelle lentille à partir des caractéristiques de la lentille préalablement identifiée, notamment l'addition, de l'âge du patient et, de mesures de surréfraction, duochrome, d'acuité visuelle en vision de près et de loin et de la détermination de l'oeil adelphe ou dominant du patient réalisées par l'utilisateur sur le patient muni de cette lentille préalablement identifiée.

Dans tous les cas (lentille sphérique, torique ou progressive), le moyen 5 de traitement de données associé audit module de calcul 51 est également agencé pour identifier l'emplacement, au sein du meuble 1, de cette nouvelle lentille.

L'invention concerne également un procédé de sélection et de réassortiment de lentilles de contact au sein d'un meuble, procédé qui est cependant avantageusement mis en oeuvre avec ce dispositif 100.

Ce procédé comporte une étape a) de stockage des données de basées sur les mesures issues d'un auto-réfractomètre 20.

Il comporte une étape b) consistant à entrer des données complémentaires relatives à la lentille de contact à sélectionner, autres que celles fournies par l'auto-réfractomètre, à savoir des données relatives à la forme de la lentille, la nature du matériau formant la lentille et la fréquence de port de cette lentille. Cette étape b) peut être réalisée au sein du dispositif, par l'intermédiaire d'une fenêtre s'affichant sur le moyen d'affichage 2 du dispositif 100. Elle peut, en variante, s'effectuer sur l'ordinateur personnel 2 de l'ophtalmologiste.

Puis, une fois lesdites données entrées par l'ophtalmologiste, ce procédé comprend une étape c) de détermination des caractéristiques de la lentille de contact sur la base d'une part, des données de mesure issues de l'auto-réfractomètre qui ont été préalablement stockées et d'autre part, des données qui ont été préalablement entrées par l'utilisateur à l'étape b).

On identifie alors, lors d'une étape d), l'emplacement, au sein du meuble 1, de la lentille de contact.

Ensuite, lors d'une étape e), on sort le boîtier contenant la lentille de contact du meuble 1. Lorsque le meuble 1 se présente sous la forme d'un distributeur automatique de boîtiers de lentilles, cette distribution est automatique si bien qu'il n'est pas nécessaire d'alerter l'ophtalmologiste, au préalable, de l'emplacement de la lentille au sein du meuble. Cela peut toutefois être envisagé.

En revanche, lorsque le meuble se présente sous la forme d'un meuble à tiroirs, tel que représenté sur la figure 1, alors on prévoit, entre les étapes d) et e), une étape additionnelle dans laquelle on alerte l'ophtalmologiste de l'emplacement de la lentille de contact au sein du meuble 1.

Une étape dans laquelle on identifie (scan) des informations contenues sur le boîtier de cette lentille de contact, lequel boîtier est muni à cet effet d'un code-barres ou d'une puce RFID.

Pour assurer le réassortiment des lentilles de contact, le procédé pourra comprendre les étapes qui suivent. On transmet alors les informations identifiées sur le boîtier de la lentille de contact à distance, par exemple par l'intermédiaire du réseau Internet, afin d'assurer le réassortiment des lentilles de contact.

Les données sur le nombre de boîtiers de lentilles de contact de mêmes caractéristiques est ainsi connu du fournisseur, au niveau du serveur distant 40. Le fournisseur peut alors décider de réaliser le réassortiment des lentilles de contact lorsqu'il le souhaite. Il peut aussi gérer ce réassortiment de manière automatique en initiant le réassortiment des lentilles de contact lorsque le nombre de boîtiers comportant des lentilles de contact identifiés et transmis à distance est en-dessous d'une valeur seuil prédéfinie.

Il convient de noter qu'en pratique, l'ophtalmologiste n'est pas dans l'obligation de mettre en oeuvre le procédé décrit précédemment. L'ophtalmologiste peut donc librement demander ou non le transfert des données depuis son ordinateur personnel 22 vers le dispositif 100.

Une étape consistant à transmettre les données caractéristiques de la lentille scannée vers l'ordinateur personnel de l'ophtalmologiste peut être envisagée. Comme expliqué précédemment, cela permet d'effectuer un suivi des lentilles de contact proposées au patient.

Par ailleurs, il peut s'avérer que la lentille issue du calcul fourni par le module de calcul 51 n'existe pas dans la grille de lentilles proposée par le fournisseur, par exemple s'il s'agit d'une lentille non standard nécessitant une fabrication sur mesure ou s'il s'agit d'une lentille de contact a priori de type « standard » ne répondant pas à l'ensemble des critères 1) à 8). Dans ce cas, l'étape c) ne permet pas de déterminer la lentille de contact dans la grille de lentilles disponibles chez le fournisseur. A l'issue des étapes a) à c), on informe alors l'utilisateur de l'absence de cette lentille. L'ophtalmologiste peut alors passer commande de celle-ci par l'intermédiaire du dispositif 100 en transmettant sa requête à distance au fournisseur.

Il convient de noter également que liaison à distance entre le dispositif 100 installé chez l'ophtalmologiste et le serveur distant 40 installé chez le fournisseur de lentilles de contact permet de gérer à distance les éventuels problèmes informatiques qui peuvent être rencontrés avec la mise en oeuvre du dispositif 100.

De même, grâce à cette liaison à distance, il est possible de réaliser une mise à jour du module de calcul 51. Cela peut être utile, par exemple, lorsque le fournisseur souhaite modifier le type de lentilles de contact « standard » présentes dans son meuble, en accord avec l'ophtalmologiste ou encore agencer ces lentilles de contact différemment, pour répondre à un souhait particulier de l'ophtalmologiste.

Enfin, comme cela a été mentionné précédemment, il est possible de réaliser un contrôlé, quelques heures ou quelque semaines après que le patient a testé la lentille de contact.

A cet effet, le procédé selon l'invention pourra comprendre, en outre, les étapes suivantes :
f) au moins réaliser une mesure de surréfraction sur un patient muni de la lentille de contact dont les caractéristiques ont été déterminées à l'étape c) ;
g) déterminer les caractéristiques d'une nouvelle lentille de contact sur la base d'une part, des données de mesures réalisée à l'étape f) et d'autre part, des caractéristiques de la lentille de contact déterminées à l'étape c) ;
h) identifier l'emplacement, au sein du meuble (1), de cette nouvelle lentille de contact ;
i) sortir du meuble (1) le boîtier comportant cette nouvelle lentille de contact.

Par ailleurs, si la lentille de contact dont les caractéristiques ont été déterminées l'étape c) est une lentille sphérique, l'étape g) consiste à déterminer la sphère, le cylindre et l'axe de la nouvelle lentille de contact.

Si la lentille de contact dont les caractéristiques ont été déterminées l'étape c) est une lentille torique, l'étape f) consiste à réaliser au moins une mesure de surréfraction et une mesure de rotation et l'étape g) consiste à déterminer la sphère, le cylindre et l'axe de la nouvelle lentille de contact

Si la lentille de contact dont les caractéristiques ont été déterminées l'étape c) est une lentille progressive, l'étape f) consiste à réaliser des mesures de surréfraction, duochrome, d'acuité visuelle en vision de près et de loin, à déterminer l'oeil adelphe ou dominant du patient et à fournir l'âge du patient et l'étape g) consiste à déterminer la sphère et/ou l'addition de la nouvelle lentille de contact.

## Revendications

1. Dispositif (100) pour sélectionner des lentilles de contact au sein d'un meuble (1) agencé pour recevoir une pluralité de lentilles de contact (50), **caractérisé en ce qu'**il comprend :
- un moyen (4) pour stocker des données basées sur les mesures issues d'un auto-réfractomètre (20);
- un moyen (5) de traitement de données associé à un module de calcul (51) pour identifier une lentille de contact sur la base d'une part, des données de issues de l'auto-réfractomètre (20) qui ont été préalablement stockées dans le moyen de stockage (4) et d'autre part, de données complémentaires relatives à la lentille de contact à identifier qui ont été préalablement entrées par l'utilisateur, ledit moyen (5) de traitement de données associé audit module de calcul (51) étant également agencé pour identifier l'emplacement, au sein du meuble (1), de cette lentille.

2. Dispositif selon la revendication 1, **caractérisé en ce qu'**il comprend un moyen pour identifier (6) les informations relatives au contenu d'un boîtier comportant la lentille de contact dont l'emplacement dans le meuble a été identifié, lequel est associé à un module (61) de prise en compte de ces données au sein du dispositif.

3. Dispositif selon la revendication précédente, **caractérisé en ce qu'**il comprend :
- un moyen (7) pour transmettre à distance, par exemple par l'intermédiaire du réseau Internet, les informations relatives à la lentille de contact dont le boîtier a été identifié.
- un serveur informatique (40) distant capable de communiquer avec le moyen (7) pour transmettre à distance ces informations relatives à la lentille de contact, le serveur (40) étant agencé pour comptabiliser les lentilles de contact sorties du meuble (1).

4. Dispositif selon la revendication précédente, **caractérisé en ce que** le module de calcul (51) est implanté dans le serveur informatique (40) distant.

5. Dispositif selon l'une des revendications 1 à 3, **caractérisé en ce que** le module de calcul (51) est logé dans le meuble (1).

6. Dispositif selon l'une des revendications précédentes, **caractérisé en ce que** le moyen (6) pour identifier les informations relatives au contenu d'un boîtier comportant la lentille de contact, par exemple un lecteur RFID ou de code-barres, est disposé à l'intérieur du meuble, avantageusement au niveau de la sortie du meuble.

7. Dispositif selon l'une des revendications précédentes, **caractérisé en ce qu'**il comprend un moyen d'affichage (2) pour afficher lesdites données complémentaires relatives à la lentille de contact parmi lesquelles des données, à fournir par un utilisateur, concernant la forme de la lentille, la nature du matériau formant la lentille et la fréquence de port de cette lentille.

8. Dispositif (100) selon l'une des revendications précédentes, **caractérisé en ce qu'**il comprend un moyen de communication (3) avec un ordinateur personnel (22) de l'utilisateur stockant les données issues de l'auto-réfractomètre, telle qu'une liaison Bluetooth®.

9. Dispositif (100) selon l'une des revendications précédentes, **caractérisé en ce que** le meuble (1) comporte plusieurs tiroirs (11), chaque tiroir (11) étant agencé pour recevoir plusieurs séries de lentilles de contact d'un même modèle pour lequel chaque série de lentilles de contact se distingue d'une autre série de lentilles de contact par la puissance de la correction ophtalmique.

10. Dispositif selon l'une des revendications précédentes, **caractérisé en ce que** le moyen (5) de traitement de données associé au module de calcul (51) est également agencé pour déterminer les caractéristiques d'une nouvelle lentille à partir des caractéristiques d'une lentille préalablement identifiée et de données complémentaires mesurées par l'utilisateur sur le patient muni de ladite lentille préalablement identifiée, ces données complémentaires comportant au moins une mesure de surréfraction, ledit moyen (5) de traitement de données associé audit module de calcul (51) étant également agencé pour identifier l'emplacement, au sein du meuble (1), de cette nouvelle lentille.

11. Dispositif selon la revendication précédente, **caractérisé en ce que**, la lentille préalablement identifiée étant une lentille sphérique, le moyen (5) de traitement de données associé au module de calcul (51) est agencé pour déterminer la sphère, le cylindre et l'axe de la nouvelle lentille à partir des caractéristiques de la lentille préalablement identifiée et d'une mesure de surréfraction réalisée sur le patient muni de cette lentille préalablement identifiée.

12. Dispositif selon l'une des revendications 10 ou 11, **caractérisé en ce que**, la lentille préalablement identifiée étant une lentille torique, le moyen (5) de traitement de données associé au module de calcul (51) est agencé pour déterminer la sphère, le cylindre et l'axe de la nouvelle lentille à partir des caractéristiques de la lentille préalablement identifiée et de mesures de surréfraction et de rotation réalisées par l'utilisateur sur le patient muni de cette lentille préalablement identifiée.

13. Dispositif selon l'une des revendications 10 à 12, **caractérisé en ce que**, la lentille préalablement identifiée étant une lentille progressive, le moyen (5) de traitement de données associé au module de calcul (51) est agencé pour déterminer la sphère et/ou l'addition de la nouvelle lentille à partir des caractéristiques de la lentille préalablement identifiée, notamment l'addition, de l'âge du patient et, de mesures de surréfraction, duochrome, d'acuité visuelle en vision de près et de loin et de la détermination de l'oeil adelphe ou dominant du patient réalisées par l'utilisateur sur le patient muni de cette lentille préalablement identifiée.

14. Procédé de sélection de lentilles de contact au sein d'un meuble, **caractérisé en ce qu'**on réalise les étapes suivantes :
a) stocker des données basées sur des mesures issues d'un auto-réfractomètre ;
b) entrer des données complémentaires relatives à la lentille de contact autres que celles fournies par l'auto-réfractomètre, à savoir des données relatives à la forme de la lentille, la nature du matériau formant la lentille et la fréquence de port de la lentille ;
c) déterminer les caractéristiques d'une lentille de contact disponible chez le fournisseur sur la base d'une part, des données de mesure issues de l'auto-réfractomètre qui ont été préalablement stockées et d'autre part, des données complémentaires préalablement entrées par l'utilisateur à l'étape b);
d) identifier l'emplacement, au sein du meuble, de cette lentille de contact ;
e) sortir du meuble le boîtier comportant cette lentille de contact.

15. Procédé selon la revendication précédente, **caractérisé en ce qu'**il comprend, en outre, les étapes suivantes :
f) au moins réaliser une mesure de surréfraction sur un patient muni de la lentille de contact dont les caractéristiques ont été déterminées à l'étape c) ;
g) déterminer les caractéristiques d'une nouvelle lentille de contact sur la base d'une part, des données de mesures réalisée à l'étape f) et d'autre part, des caractéristiques de la lentille de contact déterminées à l'étape c) ;
h) identifier l'emplacement, au sein du meuble (1), de cette nouvelle lentille de contact ;
i) sortir du meuble (1) le boîtier comportant cette nouvelle lentille de contact.

16. Procédé selon la revendication précédente, **caractérisé en ce que**, la lentille de contact dont les caractéristiques ont été déterminées l'étape c) étant une lentille sphérique, l'étape g) consiste à déterminer la sphère, le cylindre et l'axe de la nouvelle lentille de contact.

17. Procédé selon l'une des revendications 15 ou 16, **caractérisé en ce que**, la lentille de contact dont les caractéristiques ont été déterminées l'étape c) étant une lentille torique, l'étape f) consiste à réaliser au moins une mesure de surréfraction et une mesure de rotation et l'étape g) consiste à déterminer la sphère, le cylindre et l'axe de la nouvelle lentille de contact

18. Procédé selon l'une des revendications 15 à 17, **caractérisé en ce que**, la lentille de contact dont les caractéristiques ont été déterminées l'étape c) étant une lentille progressive, l'étape f) consiste à réaliser des mesures de surréfraction, duochrome, d'acuité visuelle en vision de près et de loin, à déterminer l'oeil adelphe ou dominant du patient et à fournir l'âge du patient et l'étape g) consiste à déterminer la sphère et/ou l'addition de la nouvelle lentille de contact.

19. Procédé selon la revendication précédente, **caractérisé en ce qu'**il comprend une étape pour alerter l'utilisateur de l'emplacement de la lentille de contact au sein du meuble.

20. Procédé selon l'une des revendications 14 à 16, **caractérisé en ce qu'**il comprend une étape dans laquelle on identifie des informations contenues sur le boîtier de cette lentille de contact, ledit boîtier étant muni d'un code-barres ou d'une puce RFID.

21. Procédé selon la revendication précédente, **caractérisé en ce qu'**il comprend une étape dans laquelle on transmet les informations identifiées sur le boîtier de la lentille de contact à distance, par exemple par l'intermédiaire du réseau Internet, afin d'assurer le réassortiment des lentilles de contact.

22. Procédé selon la revendication précédente, **caractérisé en ce qu'**il comprend une étape dans laquelle on initie le réassortiment des lentilles de contact lorsque le nombre de boîtiers comportant des lentilles de contact identifié et transmis à distance est en-dessous d'une valeur seuil prédéfinie.
